(19) Europäisches Patentamt / European Patent Office / Office européen des brevets

(11) **EP 4 765 134 A1**

(12) **EUROPEAN PATENT APPLICATION**

(43) Date of publication:
**24.06.2026 Bulletin 2026/26**

(21) Application number: **25195871.6**

(22) Date of filing: **14.08.2025**

(51) International Patent Classification (IPC):
**G16C 20/70** $^{(2019.01)}$    G16C 10/00 $^{(2019.01)}$
**G16C 20/10** $^{(2019.01)}$

(52) Cooperative Patent Classification (CPC):
**G16C 20/70;** G16C 10/00; G16C 20/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.12.2024 KR 20240194547**

(71) Applicant: **Samsung Electronics Co., Ltd
Suwon-si, Gyeonggi-do 16677 (KR)**

(72) Inventors:
• **SONG, Suhwan**
  **16678 Suwon-si (KR)**
• **KIM, Heejae**
  **16678 Suwon-si (KR)**
• **KIM, Geonu**
  **16678 Suwon-si (KR)**

(74) Representative: **Arnold & Siedsma
Bezuidenhoutseweg 57
2594 AC The Hague (NL)**

(54) **METHOD AND APPARATUS WITH DATA GENERATION FOR TRAINING MLFF MODEL**

(57)    A method and apparatus for generating data for training a machine-learning force field (MLFF) model are provided. The method of generating data for training the MLFF model includes generating product information based on received reactant information, generating labeled training data based on a density functional theory (DFT) calculation of the product information, identifying outlier data from verification data, based on an inference result of the verification data by the MLFF model trained based on the training data, and updating the training data based on the outlier data.

Start

Generate product information based on received reactant information — 110

Generate labeled training data based on DFT calculation of product information — 120

Identify outlier data from verification data, based on inference result of verification data of MLFF model trained based on training data — 130

Update training data based on outlier data — 140

End

FIG. 1

**Description**

BACKGROUND

1. Field

The following description relates to a method and apparatus with data generation for training a machine-learning force field (MLFF) model.

2. Description of Related Art

A machine-learning force field (MLFF) model may efficiently predict the force and energy of atoms and molecules by using machine learning. This technique has been developed to overcome the limit of conventional force field simulations and significantly reduces computational costs while maintaining a high accuracy of quantum mechanical operations. An MLFF model may be implemented to predict physical properties in a specific atomic structure by being trained with training data regarding energy and force that has been obtained through a quantum chemical operation. MLFF models are being used in various application fields, such as molecular dynamics simulation, material design, and are also used to study chemical response mechanisms.

SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

Aspects provide technology for generating the training data of a machine-learning force field (MLFF) model.

Other aspects also provide technology for automatically updating the training data for enhancing the training data to improve the inference performance of the MLFF model.

However, technical aspects are not limited to the foregoing aspects, and there may be other technical aspects.

According to a first aspect, a method as defined in any of the appended claims 1-13 is provided.

According to a second aspect, a non-transitory computer-readable storage medium as defined in appended claim 14 is provided.

According to a third aspect, an electronic device as defined in appended claim 15 is provided.

Other features and aspects will be apparent from the following detailed description, the drawings, and the claims.

BRIEF DESCRIPTION OF THE DRAWINGS

FIG. 1 illustrates a method of generating data for training a machine-learning force field (MLFF) model according to one or more embodiments.

FIG. 2 illustrates an operation of generating training data according to one or more embodiments.

FIG. 3 is a diagram illustrating a process of establishing a training data pool according to one or more embodiments.

FIGS. 4A and 4B illustrate a bond map of a snapshot based on each reaction pathway.

FIG. 5 illustrates an operation of updating training data by identifying outlier data according to one or more embodiments.

FIG. 6A illustrates an operation of determining whether each snapshot belonging to an outlier data group is a removal target according to one or more embodiments.

FIG. 6B illustrates an operation of determining whether an outlier data group is an addition target according to one or more embodiments.

FIG. 7 illustrates a data generation method according to one or more embodiments.

FIG. 8 illustrates a configuration of an electronic device according to one or more embodiments.

Throughout the drawings and the detailed description, unless otherwise described or provided, the same or like drawing reference numerals will be understood to refer to the same or like elements, features, and structures. The drawings may not be to scale, and the relative size, proportions, and depiction of elements in the drawings may be exaggerated for clarity, illustration, and convenience.

DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences of operations described herein are merely examples, and are not limited to those set forth herein, but may be changed as will be apparent after an understanding of the disclosure of this application, with the exception of operations necessarily occurring in a certain order. Also, descriptions of features that are known after an understanding of the disclosure of this application may be omitted for increased clarity and conciseness.

The features described herein may be embodied in different forms and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

The terminology used herein is for describing various examples only and is not to be used to limit the disclosure. The articles "a," "an," and "the" are intended to include the plural forms as well, unless the context clearly indicates otherwise. As used herein, the term "and/or" includes any one and any combination of any two or more of the associated listed items. As nonlimiting examples, terms "comprise" or "comprises," "include" or "includes," and "have" or "has" specify the presence of stated features, numbers, operations, members, elements, and/or combinations thereof, but do not preclude the presence or addition of one or more other features, numbers, operations, members, elements, and/or combinations thereof.

Throughout the specification, when a component or element is described as being "connected to," "coupled to," or "joined to" another component or element, it may be directly "connected to," "coupled to," or "joined to" the other component or element, or there may reasonably be one or more other components or elements intervening therebetween. When a component or element is described as being "directly connected to," "directly coupled to," or "directly joined to" another component or element, there can be no other elements intervening therebetween. Likewise, expressions, for example, "between" and "immediately between" and "adjacent to" and "immediately adjacent to" may also be construed as described in the foregoing.

Although terms such as "first," "second," and "third", or A, B, (a), (b), and the like may be used herein to describe various members, components, regions, layers, or sections, these members, components, regions, layers, or sections are not to be limited by these terms. Each of these terminologies is not used to define an essence, order, or sequence of corresponding members, components, regions, layers, or sections, for example, but used merely to distinguish the corresponding members, components, regions, layers, or sections from other members, components, regions, layers, or sections. Thus, a first member, component, region, layer, or section referred to in the examples described herein may also be referred to as a second member, component, region, layer, or section without departing from the teachings of the examples.

Unless otherwise defined, all terms, including technical and scientific terms, used herein have the same meaning as commonly understood by one of ordinary skill in the art to which this disclosure pertains and based on an understanding of the disclosure of the present application. Terms, such as those defined in commonly used dictionaries, are to be interpreted as having a meaning that is consistent with their meaning in the context of the relevant art and the disclosure of the present application and are not to be interpreted in an idealized or overly formal sense unless expressly so defined herein. The use of the term "may" herein with respect to an example or embodiment, e.g., as to what an example or embodiment may include or implement, means that at least one example or embodiment exists where such a feature is included or implemented, while all examples are not limited thereto.

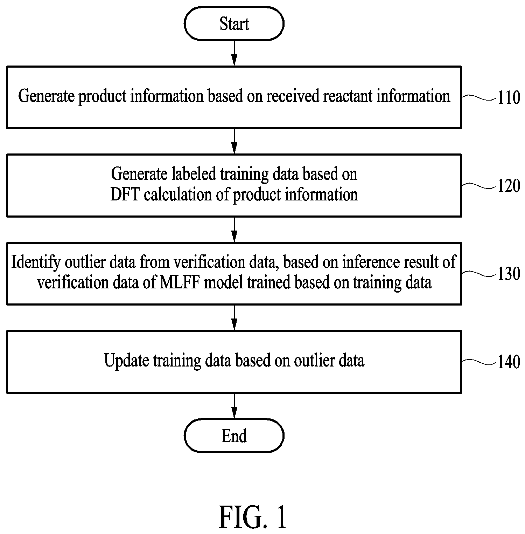

FIG. 1 illustrates a method of generating data for training a machine-learning force field (MLFF) model according to one or more embodiments.

The MLFF model may be a learning-based model for predicting force and energy between atoms (molecules or ions). Hereinafter, mention of "atoms" may be understood to also refer to molecules or ions, depending on the types of reactants or products. For example, "force between atoms" may refer to force between molecules or force between ions depending on the types of reactants or products. The method illustrated in FIG. 1 may be used to generate data used to train an MLFF model. The method of generating MLFF training data may be referred to in brief as a data generation method.

According to an embodiment, the data generation method may be performed by an electronic device. At least one processor included in the electronic device may perform the data generation method. For example, the electronic device may be a server, a terminal (e.g., a personal computer (PC), a smartphone, a tablet, a wearable device), or the like. Detailed description of an example hardware configuration of an electronic device is provided below.

Referring to FIG. 1, the data generation method may include operation 110 of generating product information based on received reactant information.

The reactant information may be input by a user and may be input or received from an external device. The reactant information specifies a reactant and may include, for example, identification of the reactant, a chemical formula of the reactant, and/or a structure of an equilibrium state of the reactant. The product information is about a material generated through a reaction of a reactant and may include, for example, information on a final product generated based on the reactant and/or information on an intermediate product (a product chronologically between the reactant and the final product).

According to an embodiment, the reactant information may be/include solid information. The solid information indicates a solid material that participates in a reaction and may include, for example, information indicating a unit cell structure. More specifically, the information indicating a unit cell structure may include, for example, a unit cell lattice vector, the Cartesian coordinates of an atom in a unit cell, and/or the direct coordinates of an atom in a unit cell.

According to an embodiment, the reactant information may be/include monomer information. The monomer information indicates a molecule that participates in a reaction and may include, for example, information indicating the chemical composition of the molecule, information indicating the arrangement and geometric structure of atoms in the molecule, information indicating the electronic structure of the molecule, information indicating the size, shape, or other physical properties of the molecule, and/or information indicating a chemical reactivity (e.g., a reaction speed constant or activation energy) of the molecule. More specifically, the monomer information may be/include the Cartesian coordinates of the atoms in the molecule and/or the internal coordinates of the atoms in the molecule. For example, the monomer information may be/include information indicating a molecule in an equilibrium state.

According to an embodiment, operation 110 of generating product information may include firstly generating a solid structure based on the solid information included in the reactant information and then generating the product information based on the solid structure. One or more solid structures may be generated based on the solid information included in the reactant information. For example, different solid structures may have the numbers of atoms, bond orders, energy distributions, angles at which atoms or molecules are bonded, and/or distances between atoms or molecules being different.

For example, the generating of a solid structure may include generating a supercell of a number of atoms in a certain range (e.g., between 150 and 250), where a bulk crystal and/or a surface structure is determined based on the solid information. Different supercells may be generated, each including the number of atoms in a certain range of a solid (e.g., iron) as indicated by the solid information. At least some of the bulk crystals and surface structures of different super cells may be different.

According to an embodiment, operation 110 of generating product information may include generating a non-solid structure based on the monomer information included in the reactant information and generating the product information based on the non-solid structure. One or more non-solid structures may be generated based on the monomer information included in the reactant information. For example, different non-solid structures may differ, between them, in their numbers of atoms, bond orders, energy distributions, angles at which atoms or molecules are bonded, and/or distances between atoms or molecules.

For example, the generating of the non-solid product may include determining a relative arrangement between atoms or molecules by using a density functional tight binding (DFTB) and/or quantum cluster growth (QCG) algorithm, based on the coordinate information of monomers included in the monomer information.

For example, the monomer information included in the reactant information may include the monomer information of a solvent and/or the monomer information of a solute. The relative arrangement between atoms or molecules by using the DFTB and/or QCG algorithm may be determined based on the coordinate information of a solvent and the coordinate information of a solute. A solvent-to-solute ratio may be variously set in the QCG algorithm. For example, the solvent-to-solute ratio may include various ratios, such as 1:1, 1:2, 1:3, and 1:4. A relative position and direction of a solvent and a solute at each ratio may be adjusted through the QCG algorithm, and a structure with a stable energy state may be determined by repeating a DFTB calculation. The non-solid structures may be generated in which at least one of the solvent-to-solute ratio, the relative position of a solvent and a solute, and the relative direction of a solvent and a solute differs among the non-solid structures.

For example, the non-solid structure may be generated as a supercell. The supercell may be generated as having the relative arrangement between molecules determined by using the DFTB and/or QCG algorithm. The supercell may be generated to have the number of atoms in a certain range (e.g., between 150 and 250). Different supercells including the number of atoms in a certain range may be generated. The number of atoms, the relative arrangement of molecules, and/or a solvent-to-solute ratio may be different among different supercells.

According to an embodiment, operation 110 of generating product information may include generating an interfacial structure of reactants included in the reactant information and generating the product information based on the interfacial structure. The interfacial structure may refer to an array of atoms, molecules, and/or ions on an interface where two or more phases or materials contact/interface. For example, the interfacial structure may include a structure on an interface where materials of different solid structures contact, a structure on an interface where materials of different non-solid structures contact, and/or a structure on an interface where a material of a solid structure contacts a material of a non-solid structure.

According to an embodiment, the generating of the interfacial structure may include generating the interfacial structure of the reactants, based on a molecular ratio of the reactants. Different ratios of reactants may be variously set in the QCG algorithm. For example, an interfacial structure having a first reactant and a second reactant at a 1:1 ratio and an interfacial structure having the first reactant and a second reactant at a 1:2 ratio by using the QCG algorithm. The first reactant may have a specific solid or non-solid structure, and the second reactant may also have a specific solid or non-solid structure.

The product information may include information indicating a product generated as a result of reaction of reactant(s). For example, the product information may include the structure of a product. The product information may be generated as a simulation result of a chemical reaction of a reactant. For example, the product information may be generated as a reactor-based (e.g., a nano-reactor) simulation result. For example, the product information may be generated based on a molecular dynamics (MD) simulation for a reactant. For example, the product information may be generated based on a meta-dynamics (MTD) simulation for a reactant. Hereafter, "the product information" may be understood as the product itself, unless the context suggests otherwise.

According to an embodiment, the product information may be generated based on a generated non-solid structure. The product information may be generated by simulating a reaction result of a reactant having a non-solid structure. For example, the product information may be generated based on a simulation result of the reactant having a non-solid structure.

According to an embodiment, the product information may be generated based on a generated interfacial structure. The product information may be generated by simulating a reaction result of an interfacial structure. For example, the product information may be generated as a simulation result of reactants in the interfacial structure.

According to an embodiment, operation 110 of generating a product structure may include generating candidate product information based on the reactant information and then storing the candidate product information in a training data pool; the storing may be based on a similarity between the candidate product information and product information stored in the training data pool. After determining whether the candidate product information generated is similar to the product information stored in the training data pool, if not similar, the candidate product information may be stored in the training data pool. On the other hand, the candidate product information that is determined to be similar to the product information stored in the training data pool may be excluded from the training data pool. The similarity between pieces of product information may be quantitatively determined by comparing at least some of the characteristics (e.g., structure) of the product indicated by the pieces of product information. For example, if the similarity between the pieces of product information is greater than or equal to a predetermined threshold value, the pieces of product information may be determined to be similar, and, if not, the pieces of product information may be determined to be unsimilar.

As noted, pieces of product information may be stored in the training data pool. A piece of product information may correspond to one reaction pathway. In other words, a product generated through one reaction pathway from reactant(s) indicated by reactant information may be one piece of reactant information. For example, n pieces of reactant information may be n pieces of product information generated through n respective reaction pathways.

According to an embodiment, the training data pool may include pieces of product information corresponding to a predetermined number of reaction pathways. The generating and storing of the candidate product information may be repeated until a number of pieces of product information matching the predetermined number of reaction pathways have been stored in the training data pool.

According to an embodiment, operation 110 of generating product information may include generating a candidate reaction pathway corresponding to the reactant information, based on an MTD simulation and storing the candidate reaction pathway in a training data pool, based on a similarity between (i) a bond map of a snapshot based on the candidate reaction pathway and (ii) a bond map of a snapshot based on a reaction pathway stored in the training data pool. This operation is described in detail below.

According to an embodiment, the data generation method may include operation 120 of generating labeled training data based on a density functional theory (DFT) calculation of the product information. The labeled training data may include product information and ground-truth (GT) data of energy and force values of the product information obtained through the DFT calculation.

The DFT calculation is used to calculate the energy and force of a material or a compound, and the energy and force values of a piece of product information (or a product indicated by the piece of product information) may be obtained through the DFT calculation of the piece of product information. For example, the DFT calculation may include ab-initio molecular dynamics (AIMD) and nudged elastic band (NEB) calculations. Since the DFT calculation is used to predict the structure of product information, the DFT calculation of the product information may be performed by using only the first step of an NEB. Three or more intermediate image structures may be generated through the NEB. An intermediate image structure may refer to an intermediate atom array or structure representing a transfer pathway between an initial state and a final state. For example, three or more intermediate image structures may be generated through the NEB. The labeled training data may be generated by performing a single-point DFT calculation of each intermediate image structure.

The training data may be used to train an MLFF model. The MLFF model may receive product information included in the training data and may be trained to output the GT data of the received product information. The trained MLFF model may output the energy and force values of product information by performing inference on the product information.

According to an embodiment, the data generation method may include operation 130 of identifying outlier data from verification data, based on an inference result of the verification data by the MLFF model trained based on the training data.

The verification data is data input to an MLFF model to verify the training provided by the training data and may include pieces of product information. The pieces of product information of the verification data may be classified into one or more data groups according to a certain criterion. For example, the one or more pieces of product information of the verification data may be classified into one or more data groups based on the number of atoms included in a product. For example, pieces of product information that indicate a respective product having 1 to 25 atoms may be placed in a first data group, and pieces of product information that indicate a respective product having 26 to 50 atoms may be placed in a second data group. For example, the pieces of product information in the verification data may be classified into data groups based on energy. For example, pieces of product information that indicate a respective product having energy between -1200 megaelectron volts (meV) and -1000 meV may be placed in the first data group, and pieces of product information that indicate a respective product having energy that between -1000 meV and -800 meV may be placed in the second data group. For example, the pieces of product information of the verification data may be classified into data groups based on the number of atoms included in a product and the energy of the product.

The outlier data is data that is determined to have a negative effect on the inference performance of an MLFF model and may include, for example, product information into which a DFT is not converged, product information of a structure physically difficult to exist, and/or product information determined to have insufficient training data.

According to an embodiment, operation 130 of identifying outlier data may include identifying the outlier data from the verification data, based on a difference between the GT data of the verification data and the inference result of the verification data produced by the MLFF model.

The inference result of the MLFF model is a result output from an MLFF corresponding to inference performed on the verification data and may include, for example, force and energy values of the product information included in the verification data. The difference between the GT data of the verification data and the value of the inference result output from the MLFF model may be referred to as an error. For example, an error metric for measuring an error may include at least one of a mean absolute error (MAE) and a mean squared error (MSE). Based on the error metric, some of the verification data may be identified as the outlier data. For example, based on the error metric, one or more of the data groups included in the verification data may be identified as outlier data groups.

The data generation method may include operation 140 of updating the training data based on the outlier data. Based on the identified outlier data, some pieces of included data may be removed from the training data. Based on the identified outlier data, some pieces of data may be added to the training data.

According to an embodiment, operation 140 of updating the training data may include determining whether the outlier data is a removal target, which may be determined based on a validity determination of the outlier data; pieces of product information corresponding to the outlier data determined to be the removal target may be removed from the training data.

According to an embodiment, operation 140 of updating the training data may include determining whether the outlier data is an addition target, based on an outlier data ratio of the inference result, and adding the training data by generating product information corresponding to the outlier data that is determined to be an addition target.

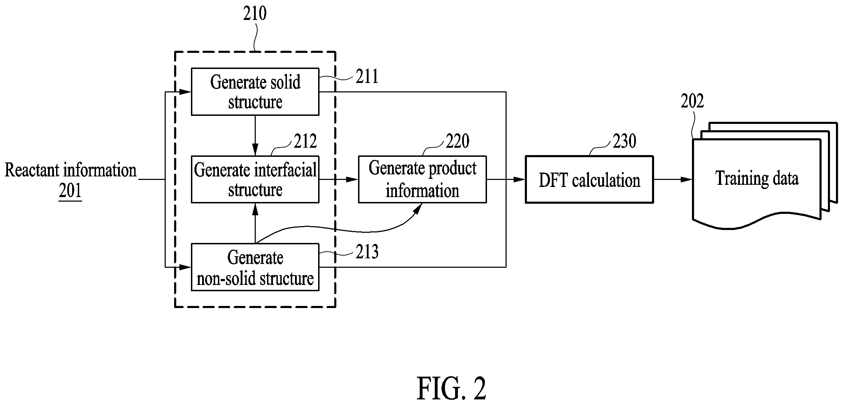

FIG. 2 illustrates an operation of generating training data according to one or more embodiments.

The training data generation operation described with reference to FIG. 2 may correspond to operations 110 and 120 of FIG. 1.

Referring to FIG. 2, the training data generation operation may include operation 210 of generating a reactant structure based on received reactant information 201. Operation 210 of generating a reactant structure may include operation 211 of generating a solid structure, operation 212 of generating an interfacial structure, and/or operation 213 of generating a non-solid structure according to the received reactant information 201.

If the received reactant information 201 includes solid information, the training data generation operation may include operation 211 of generating a solid structure. As described above, operation 211 of generating a solid structure may include generating a supercell of a number of atoms in a certain range (e.g., 150 to 250) where a bulk crystal and/or a surface structure is determined based on the solid information.

If the received reactant information 201 includes monomer information, the training data generation operation may include operation 213 of generating a non-solid structure. As described above, operation 213 of generating the non-solid product may include determining a relative arrangement between atoms or molecules by using a DFTB and/or QCG algorithm, based on the coordinate information of monomers included in the monomer information.

If structures of two or more phases or materials are generated through operation 211 and/or operation 213, the training data generation operation may include operation 212 of generating an interfacial structure. For example, operation 212 of generating an interfacial structure may include generating an interfacial structure of the generated solid and non-solid structures. For example, operation 212 of generating an interfacial structure may include generating an interfacial structure between solid structures of different materials. For example, operation 212 of generating an interfacial structure may include generating an interfacial structure between non-solid structures of different materials.

The training data generation operation may include operation 220 of generating a product structure. As described above, the product information may include the structure of a product generated as a reaction result of reactant(s). The product information may be generated as a simulation result according to reaction pathways of reactants.

The training data generation operation may include operation 230 of calculating a DFT for the generated product information. Energy and force values of the product information obtained through the DFT calculation may correspond to the GT data of the product information. Generated training data 202 may include product information labeled as GT.

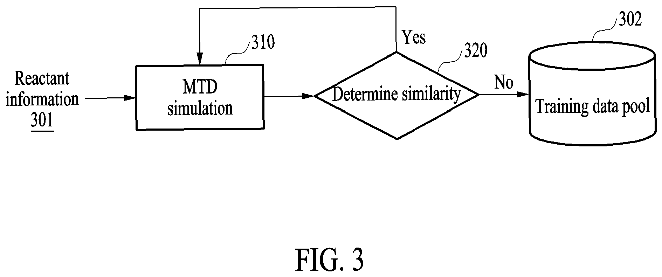

FIG. 3 illustrates a process of establishing a training data pool according to one or more embodiments.

Referring to FIG. 3, a candidate reaction pathway corresponding to reactant information 301 may be generated based on an MTD simulation 310. In other words, the candidate reaction pathway may be generated by performing the MTD simulation 310. A variance of a structure over time may be measured as a root mean square deviation (RMSD) by setting the coordinates of a reactant included in the input reactant information 301 as an initial structure. The measured RMSD may be used as collective variables (CV) of the MTD simulation 310.

For example, bias energy used in the MTD simulation 310 may be defined as shown in Equation 1.

$$\text{Equation 1}$$

$$E_{bias}^{RMSD} = \sum_{i=1}^{n} k_i \exp\left(-\alpha\Delta_i^2\right)$$

In Equation 1, n denotes the number of structures from which an RMSD is measured, $k_i$ denotes a force constant to be used in an i-th structure, $\alpha$ denotes a width of a Gaussian potential, and $\Delta_i$ denotes an RMSD of the i-th structure.

For example, $\Delta_i$ may be defined as shown in Equation 2 below.

$$\text{Equation 2}$$

$$\Delta_i = \left(\frac{1}{N}\sum_{j=1}^{N}\left(r_j - r_j^{ref,i}\right)^2\right)^{1/2}$$

For example, the MTD simulation 310 may be simulated as being performed in a nanoreactor environment. A nanoreactor may include, for example, a nanoreactor defined as a log Fermi wall potential.

For example, potential energy of a nanoreactor interface with respect to a position vector $R_A$ of a particle A may be defined as Equation 3 below.

$$\text{Equation 3}$$

$$V = \sum_A k_B T log\{1 + \exp\left[\beta(|R_A - O| - R_{sphere})\right]\}$$

In Equation 3, $k_B$ denotes a Boltzmann constant, T denotes a temperature, $\beta$ denotes a parameter that determines a gradient, and $R_{sphere}$ denotes a radius of a sphere representing the nanoreactor interface.

According to an embodiment, a temperature during the MTD simulation 310 may be set to 6000 kelvins (K), and values of $k_i$ and $\alpha$ of Equation 1 and $\beta$ of Equation 3 are determined through random sampling at every iteration of the MTD simulation 310 such that product information may be generated. The candidate reaction pathway may be determined by the values of $k_i$ and $\alpha$ of Equation 1 and $\beta$ of Equation 3. The product information may include information indicating a unique product for the candidate reaction pathway determined based on the values of $k_i$ and $\alpha$ of Equation 1 and $\beta$ of Equation 3.

A bond map of a snapshot based on a candidate reaction pathway may be generated as the result of the MTD simulation 310. The bond map of a snapshot based on a candidate reaction pathway may be generated for every candidate reaction pathway. The bond map of a snapshot based on a candidate reaction pathway may be determined by averaging internal snapshots of the candidate reaction pathway. The internal snapshots of a candidate reaction pathway may correspond to data capturing a state of a system (e.g., an atomic, molecular, or electronic structure) at a specific point of the MTD simulation 310 corresponding to the candidate reaction pathway.

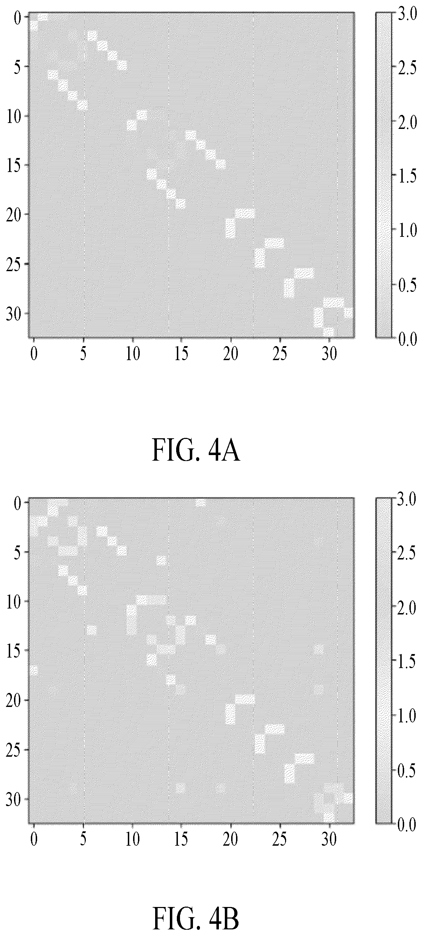

For example, FIG. 4A illustrates a bond map of a snapshot based on a first reaction pathway, and FIG. 4B illustrates a bond map of a snapshot based on a second reaction pathway. Referring to FIGS. 4A and 4B, a bond map of a snapshot may vary depending on a reaction pathway. The similarity between bond maps based on different reaction pathways may be measured based on a predetermined similarity measurement metric.

Referring to FIG. 3, the similarity between the bond map of a snapshot based on a candidate reaction pathway and the bond map of a snapshot based on a reaction pathway stored in a training data pool 302 may be determined in operation 320, and whether to store the candidate reaction pathway in the training data pool 302 may be determined based on the similarity. The storing of the candidate product in the training data pool 302 may include storing the product information obtained through the candidate reaction pathway in the training data pool 302.

The candidate reaction pathway may be omitted from inclusion in the training data pool 302 when the bond map of a snapshot based on the candidate reaction pathway is similar to the bond map of a snapshot based on a reaction pathway stored in the training data pool 302. The candidate reaction pathway may be stored in the training data pool 302 when the bond map of a snapshot based on the candidate reaction pathway is not similar to the bond map of a snapshot based on a reaction pathway stored in the training data pool 302. Sufficiency of the similarity may be determined based on whether the similarity is greater than or equal to a predetermined threshold value.

For example, pieces of product information corresponding to a predetermined number of reaction pathways may be determined to be stored in the training data pool 302. Until the pieces of product information corresponding to the predetermined number of reaction pathways are stored in the training data pool 302, product information corresponding to a candidate reaction pathway may be obtained by performing the MTD simulation 310 on the reactant information 301. In response to the pieces of product information corresponding to the predetermined number of reaction pathways being stored in the training data pool 302, the generating of product information corresponding to a new candidate reaction pathway may be terminated, and the labeling of training data based on a DFT calculation of product information corresponding to a reaction pathway stored in the training data pool 302 may be performed.

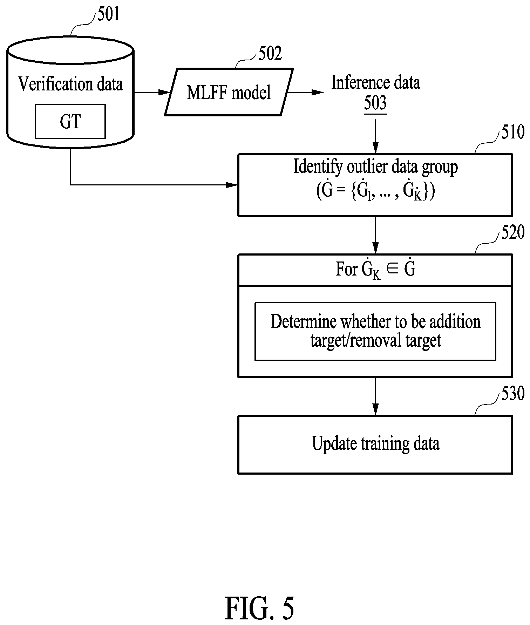

FIG. 5 illustrates an operation of updating training data by identifying outlier data according to one or more embodiments.

As described above, verification data 501 may be input to a trained MLFF model 502. The verification data 501 may include one or more data groups. A data group included in the verification data 501 may include one or more snapshots. A snapshot may correspond to a reaction pathway.

For example, a kth data group $G_k$ ($1 \leq k \leq K$) included in the verification data 501 may be defined as two-dimensional (2D) binning as shown in Equation 4 below.

$$\text{Equation 4}$$

$$G_k = \{s_i \in D_{valid} | n_k \leq \pi_i < n_{k+1}, e_k \leq E_i < e_{k+1}\}$$

In Equation 4, $s_i$ denotes an i-th snapshot, $D_{valid}$ denotes the verification data 501, $\pi_i$ denotes the number of atoms included by $s_i$, and $E_i$ denotes an energy value (meV) included by the GT data of $s_i$. In other words, the data group $G_k$ may be a data group generated by classifying snapshots based on the number of atoms and energy.

In addition, $n_k$ and $e_k$ may be defined as shown in Equations 5 and 6 below, respectively.

$$\text{Equation 5}$$

$$n_k = \frac{(k-1) \max\limits_{s_i \in D_{valid}} \pi_i}{K}$$

$$\text{Equation 6}$$

$$e_k = \frac{(k-1) \max\limits_{s_i \in D_{valid}} -E_i}{K}$$

For example, if K = 10, max $\pi_i$ = 100, and max $-E_i$ = 2000, $G_3$ may be a group of snapshots with the number of atoms being greater than or equal to 20 and less than 30 and energy being greater than or equal to -600 meV and less than -400 meV.

According to an embodiment, based on an inference result 503 of the verification data 501 by the MLFF model 502 and/or GT data included in the verification data 501, some of data groups included in the verification data 501 may be identified as outlier data groups in operation 510.

In an example, when $\mu(s_i)$ is an error metric of $s_i$, and $\beta_0$ is an outlier threshold, an outlier data group may be defined as a data group including one or more snapshots which are $\mu(s_i) > \beta_0$ among $G_1, ..., G_K$. As described above, an error metric is used to measure the difference between GT data and the value of an inference result output from the MLFF model and may include, for example, an MAE value or an MSE value of the inference result and the GT data.

An outlier data group may be expressed as shown in Equation 7 below.

Equation 7

$$\dot{G} = \{\dot{G}_1, \ldots, \dot{G}_{\dot{K}}\}$$

The number $\dot{K}$ of elements of the outlier data group included in an outlier data group set may be less than the number K of data groups included in a data group G. An outlier data group set $\dot{G}$ may be aligned in a descending order with respect to an outlier data ratio $r_\kappa$. $r_\kappa$ may be defined as shown in Equation 8.

Equation 8

$$r_\kappa = \frac{|s_i \in \dot{G}_\kappa : \mu(s_i) > \beta_0|}{|s_i \in \dot{G}_\kappa|}$$

In Equation 8, the vertical-bars symbol "||" denotes the number of elements included in a set.

According to an embodiment, for each outlier data group, whether such a data group is an addition target and/or a removal target may be determined in operation 520. Determining whether each outlier data group is an addition target and/or a removal target is described in detail below.

Training data may be updated in operation 530 based on a determination result of whether each outlier data group is an addition target and/or a removal target. For example, information indicating a reaction pathway corresponding to an outlier data group determined to be the removal target may be obtained. Product information corresponding to a reaction pathway obtained from a training data pool may be removed. For example, information indicating a reaction pathway corresponding to an outlier data group determined to be the addition target may be obtained. By performing an MTD simulation corresponding to the obtained reaction pathway, product information corresponding to the reaction pathway may be generated to be stored in the training data pool.

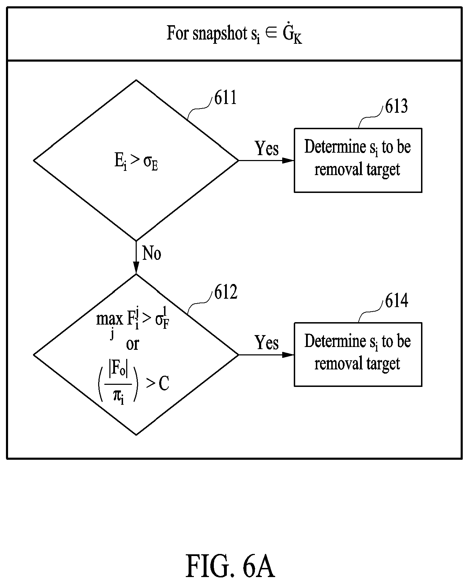

FIG. 6A illustrates an operation of determining whether each snapshot $s_i$ belonging to an outlier data group $\dot{G}_\kappa$ is a removal target according to one or more embodiments.

Referring to FIG. 6A, determining whether a snapshot $s_i$ belonging to the outlier data group $\dot{G}_\kappa$ is a removal target may include determining the validity of a GT energy value $E_i$ of $s_i$. The GT energy value $E_i$ may be data included in the GT data of $s_i$. The validity of $E_i$ may be determined in operation 611 by whether the GT energy value $E_i$ of $s_i$ exceeds a threshold energy value $\sigma_E$. When $E_i$ of $s_i$ exceeds $\sigma_E$, $s_i$ may be determined to be a removal target in operation 613. When $E_i$ exceeds $\sigma_E$, $E_i$ may be determined to be invalid. In other words, when $E_i$ exceeds $\sigma_E$, it may be determined that a pseudo-potential used for a DFT calculation has exceeded an expressible energy range and there has been an error in the DFT calculation (i.e., is invalid).

Determining whether a snapshot $s_i$ belonging to the outlier data group $\dot{G}_\kappa$ is a removal target may include determining the validity of a GT force value $F_i^j$ of an atom $a_j$ included in the snapshot $s_i$. The GT force value $F_i^j$ may be data included in the GT data of $s_i$, which is $F_i^j = \sqrt{F_i^{j,x^2} + F_i^{j,y^2} + F_i^{j,z^2}}$. The determining of the validity of the GT force value $F_i^j$ of the atom $a_j$ included in the snapshot $s_i$ may be performed when $E_i$ is determined to not exceed $\sigma_E$.

The validity of $F_i^j$ may be determined in operation 612 by whether a maximum value $\max_j F_i^j$ of a GT force value of an atom included in the snapshot $s_i$ exceeds a first threshold force value $\sigma_F^1$, and, a ratio of the number of atoms included in the snapshot $s_i$ to the number $|F_o|$ of atoms of $s_i$ having a GT force value greater than a second threshold force value $\sigma_F^2$, which is $F_o = \{a_j | F_i^j > \sigma_F^2\}$, and $|F_o|$ may be the number of elements included in $F_o$. $\pi_i$ may be the number of atoms included in the snapshot $s_i$. When (i) the maximum value $\max_j F_i^j$ of the GT force value of the atom is greater than the first threshold force value $\sigma_F^1$ and (ii) the ratio of the number of atoms included in the snapshot $s_i$ to the number $|F_o|$ of atoms of $s_i$ having the GT force value greater than the second threshold force value $\sigma_F^2$ is greater than c, $s_i$ may be determined to be a removal target in operation 614. Because the atom having a force value greater than the first threshold force value $\sigma_F^1$ has excessive repulsive force between nuclei, the force value of the atom may be determined to be invalid. Because the number of atoms having the force value greater than $\sigma_F^2$ being greater than c is determined to be a result of the non-convergence of a DFT due to the non-convergence of a self-consistent field (SCF) or an insufficient K-point used for a DFT calculation, the force value of the atom may be determined to be invalid.

When the maximum value $\max_j F_i^j$ of the GT force value of the atom is less than the first threshold force value $\sigma_F^1$, and the ratio of the number of atoms included in the snapshot $s_i$ to the number $|F_o|$ of atoms of $s_i$ having the GT force value greater than the second threshold force value $\sigma_F^2$ is less than or equal to c, $s_i$ may be determined to not be a removal target.

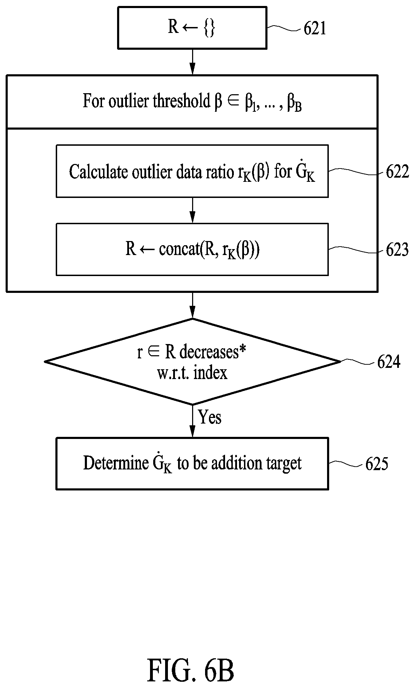

FIG. 6B illustrates an operation of determining whether an outlier data group $\dot{G}_\kappa$ is an addition target, according to one or more embodiments.

Referring to FIG. 6B, a set R for the outlier data group $\dot{G}_\kappa$ may be initialized to a null set in operation 621.

B relaxed outlier threshold values $\beta_1$, $\beta_2$, ..., $\beta_B$ may be determined. The B relaxed outlier threshold values may satisfy $\beta_1 < \beta_2 < ... < \beta_B$.

An outlier data ratio for the outlier data group $\dot{G}_\kappa$ may be calculated in operation 622 as shown in Equation 9 with respect to an outlier threshold value $\beta \in \beta_1, \beta_2, ..., \beta_B$ by rewriting Equation 8.

Equation 9

$$r_\kappa(\beta) = \frac{|s_i \in \dot{G}_\kappa : \mu(s_i) > \beta|}{|s_i \in \dot{G}_\kappa|}$$

$r_\kappa(\beta)$ calculated for the outlier threshold value $\beta \in \beta_1, \beta_2, ..., \beta_B$ may be stored in R in operation 623. R = $(r_\kappa(\beta_1), ..., r_\kappa((\beta_B))$ including a calculation result for each of $\beta_1$ , ..., $\beta_B$ may be obtained.

Whether R for the outlier data group $\dot{G}_\kappa$ is an index, that is, a monotone decreasing function for 1, ..., B may be determined in operation 624. If R for the outlier data group is an index, that is, a monotone decreasing function for 1, ..., B, the number of snapshots of the outlier data group $\dot{G}_\kappa$ included in the training data of the MLFF model is determined to be small. Accordingly, the outlier data group $\dot{G}_\kappa$ may be determined to be an addition target in operation 625. In other words, when R for the outlier data group $\dot{G}_\kappa$ is $r_\kappa(\beta_1) \geq r_K((\beta_2) \geq ... \geq r_\kappa(\beta_B)$, and $r_\kappa(\beta_B) < 1.0$, the outlier data group $\dot{G}_\kappa$ may be determined to be an addition target in operation 625.

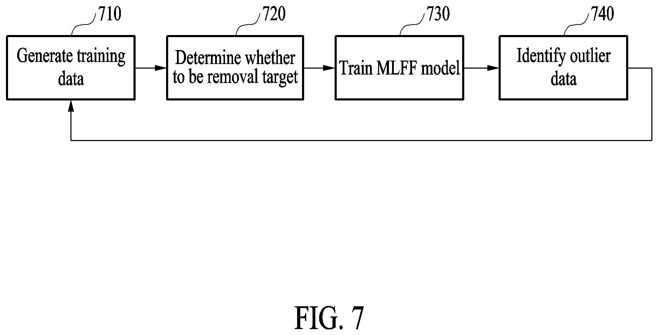

FIG. 7 illustrates a data generation method according to one or more embodiments.

Referring to FIG. 7, operation 720 of determining whether an outlier data group is a removal target may be performed before operation 730 of training an MLFF model based on generated training data.

For example, operation 720 of determining whether an outlier data group included in the training data is a removal target may be performed after operation 710 of generating the training data. Whether to be a removal target is determined based on GT data. Thus, whether data corresponding to the removal target is included in the generated training data may be determined.

The MLFF model may be trained based on the training data from which the data determined to be a removal target has been removed.

Operation 740 of identifying outlier data may include determining an identified outlier data group is an addition target, based on the difference between verification data and an inference result output from the trained MLFF model for the verification data.

Product information corresponding to a reaction pathway corresponding to the outlier data group determined to be the addition target may be additionally generated to be added to the training data.

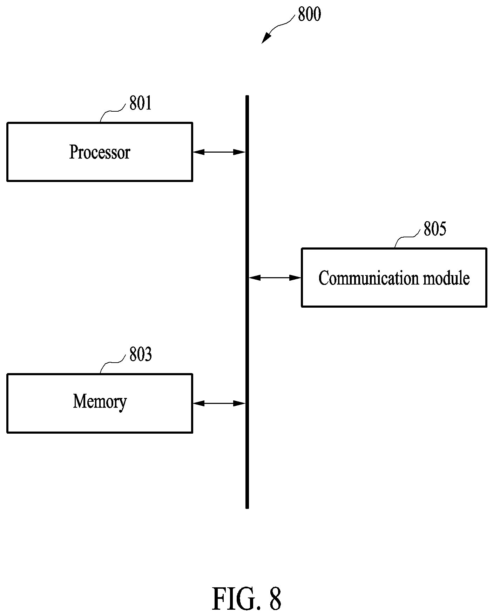

FIG. 8 illustrates a configuration of an electronic device according to one or more embodiments.

Referring to FIG. 8, an electronic device 800 may include a processor 801, a memory 803, and a communication module 805. The electronic device 800 may include at least one of, for example, a server and a terminal (e.g., a PC, a smartphone, a tablet, or a wearable device) as a device for performing the data generation method described above with reference to FIGS. 1 to 7.

The processor 801 may perform at least one operation of the data generation method described above with reference to FIGS. 1 to 7. For example, the processor 801 may perform at least one of generating product information based on received reactant information, generating labeled training data based on a DFT calculation of the product information, identifying outlier data from verification data, based on an inference result of the verification data of an MLFF model trained based on the training data, and updating the training data based on the outlier data.

The memory 803 may be a volatile or non-volatile memory and may store data regarding the data generation method described above with reference to FIGS. 1 to 7. For example, the memory 803 may store data generated in a process of performing the data generation method or data necessary for performing the data generation method. For example, the memory 803 may store product information generated based on reactant information and GT data obtained through a DFT calculation of the product information. For example, the memory 803 may include a training data pool.

The communication module 805 may provide a function for the electronic device 800 to communicate with other electronic devices or other servers over a network. In other words, the electronic device 800 may be connected to an external device (e.g., a terminal of a user, a server, or a network) via the communication device 805 and may exchange data.

The memory 803 may not be a component of the electronic device 800 but may be included in the external device accessible from the electronic device 800. In this case, the electronic device 800 may receive data stored in the memory 803 included in the external device and may transmit data to be stored in the memory 803 via the communication module 805.

The memory 803 may store a program implementing the data generation method described above with reference to FIGS. 1 to 7. The processor 801 may execute the program stored in the memory 803 and may control the electronic device 800. Code of the program executed by the processor 801 may be stored in the memory 803.

The memory 803 may store instruction(s). The instruction(s) stored in the memory 803, when executed by the processor 801, may cause the electronic device 800 to generate product information based on received reactant information, generate labeled training data based on a DFT calculation of the product information, identify outlier data from verification data, based on an inference result of the verification data by the MLFF model trained based on the training data, and update the training data based on the outlier data.

The electronic device 800 may further include components not shown in the drawings. For example, the electronic device 800 may further include an input/output interface including an input device and an output device as the means of interfacing with the communication module 805. For another example, the electronic device 800 may further include other components, such as a transceiver, various sensors, and a database.

The computing apparatuses, the electronic devices, the processors, the memories, the displays, the information output system and hardware, the storage devices, and other apparatuses, devices, units, modules, and components described herein with respect to FIGS. 1-8 are implemented by or representative of hardware components. Examples of hardware components that may be used to perform the operations described in this application where appropriate include controllers, sensors, generators, drivers, memories, comparators, arithmetic logic units, adders, subtractors, multipliers, dividers, integrators, and any other electronic components configured to perform the operations described in this application. In other examples, one or more of the hardware components that perform the operations described in this application are implemented by computing hardware, for example, by one or more processors or computers. A processor or computer may be implemented by one or more processing elements, such as an array of logic gates, a controller and an arithmetic logic unit, a digital signal processor, a microcomputer, a programmable logic controller, a field-programmable gate array, a programmable logic array, a microprocessor, or any other device or combination of devices that is configured to respond to and execute instructions in a defined manner to achieve a desired result. In one example, a processor or computer includes, or is connected to, one or more memories storing instructions or software that are executed by the processor or computer. Hardware components implemented by a processor or computer may execute instructions or software, such as an operating system (OS) and one or more software applications that run on the OS, to perform the operations described in this application. The hardware components may also access, manipulate, process, create, and store data in response to execution of the instructions or software. For simplicity, the singular term "processor" or "computer" may be used in the description of the examples described in this application, but in other examples multiple processors or computers may be used, or a processor or computer may include multiple processing elements, or multiple types of processing elements, or both. For example, a single hardware component or two or more hardware components may be implemented by a single processor, or two or more processors, or a processor and a controller. One or more hardware components may be implemented by one or more processors, or a processor and a controller, and one or more other hardware components may be implemented by one or more other processors, or another processor and another controller. One or more processors, or a processor and a controller, may implement a single hardware component, or two or more hardware components. A hardware component may have any one or more of different processing configurations, examples of which include a single processor, independent processors, parallel processors, single-instruction single-data (SISD) multiprocessing, single-instruction multiple-data (SIMD) multiprocessing, multiple-instruction single-data (MISD) multiprocessing, and multiple-instruction multiple-data (MIMD) multiprocessing.

The methods illustrated in FIGS. 1-8 that perform the operations described in this application are performed by computing hardware, for example, by one or more processors or computers, implemented as described above implementing instructions or software to perform the operations described in this application that are performed by the methods. For example, a single operation or two or more operations may be performed by a single processor, or two or more processors, or a processor and a controller. One or more operations may be performed by one or more processors, or a processor and a controller, and one or more other operations may be performed by one or more other processors, or another processor and another controller. One or more processors, or a processor and a controller, may perform a single operation, or two or more operations.

Instructions or software to control computing hardware, for example, one or more processors or computers, to implement the hardware components and perform the methods as described above may be written as computer programs, code segments, instructions or any combination thereof, for individually or collectively instructing or configuring the one or more processors or computers to operate as a machine or special-purpose computer to perform the operations that are performed by the hardware components and the methods as described above. In one example, the instructions or software include machine code that is directly executed by the one or more processors or computers, such as machine code produced by a compiler. In another example, the instructions or software includes higher-level code that is executed by the one or more processors or computer using an interpreter. The instructions or software may be written using any programming language based on the block diagrams and the flow charts illustrated in the drawings and the corresponding descriptions herein, which disclose algorithms for performing the operations that are performed by the hardware components and the methods as described above.

The instructions or software to control computing hardware, for example, one or more processors or computers, to implement the hardware components and perform the methods as described above, and any associated data, data files, and data structures, may be recorded, stored, or fixed in or on one or more non-transitory computer-readable storage media. Examples of a non-transitory computer-readable storage medium include read-only memory (ROM), random-access programmable read only memory (PROM), electrically erasable programmable read-only memory (EEPROM), random-access memory (RAM), dynamic random access memory (DRAM), static random access memory (SRAM), flash memory, non-volatile memory, CD-ROMs, CD-Rs, CD+Rs, CD-RWs, CD+RWs, DVD-ROMs, DVD- Rs, DVD+Rs, DVD-RWs, DVD+RWs, DVD-RAMs, BD-ROMs, BD-Rs, BD-R LTHs, BD-REs, blue-ray or optical disk storage, hard disk drive (HDD), solid state drive (SSD), flash memory, a card type memory such as a multimedia card or a micro card (for example, secure digital (SD) or extreme digital (XD)), magnetic tapes, floppy disks, magneto-optical data storage devices, optical data storage devices, hard disks, solid-state disks, and any other device that is configured to store the instructions or software and any associated data, data files, and data structures in a non-transitory manner and provide the instructions or software and any associated data, data files, and data structures to one or more processors or computers so that the one or more processors or computers can execute the instructions. In one example, the instructions or software and any associated data, data files, and data structures are distributed over network-coupled computer systems so that the instructions and software and any associated data, data files, and data structures are stored, accessed, and executed in a distributed fashion by the one or more processors or computers.

The present disclosure may also relate to any of the following clauses:

Clause 1. A method, performed by one or more processors, of generating data for training a machine-learning force field (MLFF) model, the method comprising: generating product information based on received reactant information; generating labeled training data based on a density functional theory (DFT) calculation of the product information; identifying outlier data from verification data, wherein the identifying is based on an inference result of performing inference on the verification data by the MLFF model as trained based on the labeled training data; and updating the labeled training data based on the outlier data.

Clause 2. The method of clause 1, wherein the reactant information comprises at least one of solid information and monomer information.

Clause 3. The method of clause 1 or 2, wherein the product information represents a product of a reaction of a reactant represented by the reactant information, and wherein the generating the product information comprises: generating candidate product information based on the reactant information; and storing the candidate product information in a training data pool, wherein the storing is based on a similarity between the candidate product information and product information stored in the training data pool.

Clause 4. The method of any of the clauses 1-3, wherein the generating the product information comprises: generating a candidate reaction pathway corresponding to the reactant information, based on a meta-dynamics (MTD) simulation; and storing the candidate reaction pathway in a training data pool, based on a similarity between a bond map of a snapshot based on the candidate reaction pathway and a bond map of a snapshot based on a reaction pathway stored in the training data pool.

Clause 5. The method of clause 4, wherein the training data pool comprises pieces of product information corresponding to a predetermined number of reaction pathways.

Clause 6. The method of any of the clauses 1-5, wherein the identifying the outlier data comprises: identifying the outlier data from the verification data, based on a difference between ground-truth data of the verification data and the inference result of the inference performed on the verification data by the MLFF model.

Clause 7. The method of any of the clauses 1-6, wherein the updating the training data comprises: determining whether the outlier data is a removal target, based on a validity determination of the outlier data; and removing, from the training data, product information corresponding to the outlier data determined to be the removal target.

Clause 8. The method of any of the clauses 1-7, wherein the updating the training data comprises: determining whether the outlier data is an addition target, based on a ratio of outlier data of the inference result; and adding the training data by generating product information corresponding to the outlier data determined to be an addition target.

Clause 9. The method of any of the clauses 1-8, wherein the generating the product information comprises: generating an interfacial structure of reactants comprised in the reactant information; and generating the product information based on the interfacial structure.

Clause 10. The method of clause 9, wherein the generating the interfacial structure comprises: generating the interfacial structure of the reactants, based on a molecular ratio of the reactants.

Clause 11. The method of any of the clauses 1-10, wherein the generating the product information comprises: generating a solid structure based on solid information comprised in the reactant information; and generating the product information based on the solid structure.

Clause 12. The method of any of the clauses 1-11, wherein the generating the product information comprises: generating a non-solid structure based on monomer information comprised in the reactant information; and generating the product information based on the non-solid structure.

Clause 13. A non-transitory computer-readable storage medium storing instructions that, when executed by a processor, cause the processor to perform the method of any of the clauses 1-12.

Clause 14. An electronic device comprising: one or more processors; and a memory storing instructions configured to cause the one or more processors to: generate product information based on received reactant information, generate labeled training data based on a density functional theory (DFT) calculation of the product information, identify outlier data from verification data, wherein the identifying is based on an inference result of performing inference on the verification data of a machine-learning force field (MLFF) model as trained based on the labeled training data, and update the labeled training data based on the outlier data.

Clause 15. The electronic device of clause 14, wherein the product information represents a product of a reaction of a reactant represented by the reactant information, and wherein the reactant information comprises at least one of solid information and monomer information.

Clause 16. The electronic device of clause 14 or 15, wherein the generating the product information comprises: generating candidate product information based on the reactant information; and storing the candidate product information in a training data pool, wherein the storing is based on a similarity between the candidate product information and product information stored in the training data pool.

Clause 17. The electronic device of any of the clauses 14-16, wherein the generating the product information comprises: generating a candidate reaction pathway corresponding to the reactant information, based on a meta-dynamics (MTD) simulation; and storing the candidate reaction pathway in a training data pool, based on a similarity between a bond map of a snapshot based on the candidate reaction pathway and a bond map of a snapshot based on a reaction pathway stored in the training data pool.

Clause 18. The electronic device of any of the clauses 14-17, wherein the identifying the outlier data comprises: identifying the outlier data from the verification data, based on a difference between ground-truth data of the verification data and the inference result of the inference performed on the verification data by the MLFF model.

Clause 19. The electronic device of any of the clauses 14-18, wherein the generating the product information comprises: generating an interfacial structure of reactants comprised in the reactant information; and generating the product information based on the interfacial structure.

Clause 20. The electronic device of clause 19, wherein the generating the interfacial structure comprises: generating the interfacial structure of the reactants, based on a molecular ratio of the reactants.

While this disclosure includes specific examples, it will be apparent after an understanding of the disclosure of this application that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components.

**Claims**

1. A method, performed by one or more processors, of generating data for training a machine-learning force field, 'MLFF', model, the method comprising:

generating product information based on received reactant information;

generating labeled training data based on a density functional theory, 'DFT', calculation of the product information;

identifying outlier data from verification data, wherein the identifying is based on an inference result of performing inference on the verification data by the MLFF model as trained based on the labeled training data; and

updating the labeled training data based on the outlier data.

2. The method of claim 1, wherein the reactant information comprises at least one of solid information and monomer information.

3. The method of claim 1 or 2, wherein the product information represents a product of a reaction of a reactant represented by the reactant information.

4. The method of any of the previous claims, wherein generating the product information comprises:

generating candidate product information based on the reactant information; and

storing the candidate product information in a training data pool, wherein the storing is based on a similarity between the candidate product information and product information stored in the training data pool.

5. The method of any of the previous claims, wherein generating the product information comprises:

generating a candidate reaction pathway corresponding to the reactant information, based on a meta-dynamics, 'MTD', simulation; and

storing the candidate reaction pathway in a training data pool, based on a similarity between a bond map of a snapshot based on the candidate reaction pathway and a bond map of a snapshot based on a reaction pathway stored in the training data pool.

6. The method of claim 5, wherein the training data pool comprises pieces of product information corresponding to a predetermined number of reaction pathways.

7. The method of any of the previous claims, wherein identifying the outlier data comprises:
identifying the outlier data from the verification data, based on a difference between ground-truth data of the verification data and the inference result of the inference performed on the verification data by the MLFF model.

8. The method of any of the previous claims, wherein updating the training data comprises:

determining whether the outlier data is a removal target, based on a validity determination of the outlier data; and

removing, from the training data, product information corresponding to the outlier data determined to be the removal target.

9. The method of any of the previous claims, wherein updating the training data comprises:

determining whether the outlier data is an addition target, based on a ratio of outlier data of the inference result; and

adding the training data by generating product information corresponding to the outlier data determined to be an addition target.

10. The method of any of the previous claims, wherein generating the product information comprises:

generating an interfacial structure of reactants comprised in the reactant information; and

generating the product information based on the interfacial structure.

11. The method of claim 10, wherein generating the interfacial structure comprises:
generating the interfacial structure of the reactants, based on a molecular ratio of the reactants.

12. The method of any of the previous claims, wherein generating the product information comprises:

generating a solid structure based on solid information comprised in the reactant information; and

generating the product information based on the solid structure.

13. The method of any of the previous claims, wherein generating the product information comprises:

generating a non-solid structure based on monomer information comprised in the reactant information; and generating the product information based on the non-solid structure.

14. A non-transitory computer-readable storage medium storing instructions that, when executed by a processor, cause the processor to perform the method of any of the previous claims.

15. An electronic device, comprising:

one or more processors; and
a memory storing instructions configured to cause the one or more processors to perform the method of any of the claims 1-13.

Start

Generate product information based on received reactant information ⟩~110

Generate labeled training data based on
DFT calculation of product information ⟩~120

Identify outlier data from verification data, based on inference result of
verification data of MLFF model trained based on training data ⟩~130

Update training data based on outlier data ⟩~140

End

FIG. 1

FIG. 2

Reactant
information
301
→ MTD
simulation
/310
→ Determine similarity
/320
Yes
No
→ Training data pool
/302

FIG. 3

EP 4 765 134 A1

FIG. 4A

FIG. 4B

19

501

Verification data

GT

502

MLFF model

Inference data
503

510

Identify outlier data group
$(\dot{G} = \{\dot{G}_1, \ldots, \dot{G}_{\dot{K}}\})$

520

For $\dot{G}_K \in \dot{G}$

Determine whether to be addition
target/removal target

530

Update training data

FIG. 5

For snapshot $s_i \in \dot{G}_K$

$E_i > \sigma_E$ /611

Yes → Determine $s_i$ to be removal target /613

No ↓

$\max_j F_i^j > \sigma_F^1$
or
$\left( \dfrac{|F_o|}{\pi_i} \right) > C$ /612

Yes → Determine $s_i$ to be removal target /614

# FIG. 6A

R ← {} — 621

For outlier threshold $\beta \in \beta_1, \ldots, \beta_B$

Calculate outlier data ratio $r_K(\beta)$ for $\dot{G}_K$ — 622

R ← concat(R, $r_K(\beta)$) — 623

$r \in R$ decreases*
w.r.t. index — 624

Yes

Determine $\dot{G}_K$ to be addition target — 625

FIG. 6B

710

Generate training data

720

Determine whether to be removal target

730

Train MLFF model

740

Identify outlier data

FIG. 7

800

801

Processor

805

Communication module

803

Memory

FIG. 8

**EUROPEAN SEARCH REPORT**

Europäisches
Patentamt
European
Patent Office
Office européen
des brevets

Application Number

EP 25 19 5871

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOTU V. ET AL: "Machine Learning Force Fields: Construction, Validation, and Outlook", THE JOURNAL OF PHYSICAL CHEMISTRY C, vol. 121, no. 1, 29 December 2016 (2016-12-29), pages 511-522, XP093357648, US ISSN: 1932-7447, DOI: 10.1021/acs.jpcc.6b10908 Retrieved from the Internet: URL:http://pubs.acs.org/doi/pdf/10.1021/acs.jpcc.6b10908> * the whole document * | 1-15 | INV. G16C20/70 ADD. G16C10/00 G16C20/10 |
| X | UNKE OLIVER T. ET AL: "Machine Learning Force Fields", CHEMICAL REVIEWS, vol. 121, no. 16, 11 March 2021 (2021-03-11), pages 10142-10186, XP093358256, ISSN: 0009-2665, DOI: 10.1021/acs.chemrev.0c01111 Retrieved from the Internet: URL:https://pubs.acs.org/doi/pdf/10.1021/acs.chemrev.0c01111> * the whole document * | 1-15 | TECHNICAL FIELDS SEARCHED (IPC) G16C |

The present search report has been drawn up for all claims

| Place of search | Date of completion of the search | Examiner |
|---|---|---|
| The Hague | 28 January 2026 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)